# EUROPEAN PATENT SPECIFICATION

(11) **EP 1 765 144 B1**
(45) Date of publication and mention of the grant of the patent: **18.11.2015**
(21) Application number: 05756914.7
(22) Date of filing: 30.06.2005
(51) Int. Cl.: A61B 1/00, A61B 1/04

(54) **IN-VIVO SENSING SYSTEM DEVICE AND METHOD FOR REAL TIME VIEWING**
IN-VIVO-NACHWEISSYSTEM UND VERFAHREN FÜR DIE ECHTZEIT-ANSICHT
SYSTEME ET PROCEDE DE DETECTION IN VIVO POUR VISUALISATION EN TEMPS REEL

(30) Priority: 30.06.2004 US 583882 P; 01.04.2005 US 667074 P
(43) Date of publication of application: 28.03.2007
(73) Proprietor: Given Imaging Ltd., 20692 Yoqneam (IL)
(72) Inventor: NISANI, Micha, 36781 Nesher (IL); PASCAL, Pesach, 36841 Nesher (IL); DAVIDSON, Tal, 20692 Yoqneam Illite (IL); RUBEY, Kevin, Ventura, California 93003 (US); HORN, Eli, 26315 Kiryat Motzkin (IL); SKALA, Michael, 30900 Zichron Yaaqov (IL)
(74) Representative: Hoffmann Eitle
(86) International application number: PCT/IL2005/000696
(87) International publication number: WO 2006/003650

(56) References cited:
- EP-A- 0 677 272
- WO-A-03/094723
- US-A1- 2001 051 766
- US-A1- 2002 067 408
- US-A1- 2002 067 408
- US-A1- 2003 151 661
- US-A1- 2003 167 000
- US-A1- 2004 176 685

## Description

### FIELD OF THE INVENTION

The present invention relates to in vivo imaging. More specifically the invention relates to an apparatus and method for concurrent receiving, recording, processing and presenting information gathered by an in-vivo sensing device.

### BACKGROUND OF THE INVENTION

In-vivo devices, such as, for example, capsules having image capturing capabilities, may transmit streams of images while progressing through body lumens. Such a stream of images may be recorded in a memory of a recording device and may be used by human operators as, for example, a source of information regarding the health condition of such body lumens. Document US2004/0176685 A1 discloses a capsule medical system. Document US2003/0151661 A1 discloses a system and method for displaying an image stream.

### SUMMARY OF THE INVENTION

There is provided, in accordance with some embodiments of the present invention an in-vivo sensing system which may include an in-vivo sensing device, such as, for example, a capsule having image capturing capabilities, and a receiver/recorder, to receive information, for example, a stream of images from the in-vivo sensing device and to store the stream of images in a memory for a later use.

In addition, the in-vivo sensing system may include a workstation and/or a portable device, capable of downloading the stream of images from the receiver/recorder and capable of processing and/or analyzing and/or displaying the stream of images, for example in real time. According to some embodiments of the present invention, the information may be downloaded, for example from the receiver/recorder to a portable memory in real time.

Moreover, according to some embodiments of the present invention, the receiver/recorder may be capable of recording the information from the sensing device, for example to a memory, while simultaneously accepting input from the sensing device. An associated workstation and/or portable device may be capable of controlling the receiver/recorder to download selected images in a selected order while the receiver/recorder is recording other images of the stream of images.

Embodiments of the invention are illustrated by way of example and not limitation in the figures of the accompanying drawings, in which like reference numerals indicate corresponding, analogous or similar elements, and in which:
Fig. 1A is a simplified illustration of an exemplary in-vivo sensing system, including a sensing device and a receiver/recorder, in accordance with some embodiments of the present invention;
Fig. 1B is a simplified illustration of an exemplary in-vivo sensing system, including a sensing device, and a receiver/recorder, a workstation, a portable device and a portable memory, in accordance with some embodiments of the present invention;
Fig. 1C is an exemplary Toolbox screen, in accordance with some embodiments of the present invention;
Fig. 2 is an exemplary simplified block-diagram illustration of the in-vivo sensing system, in accordance with some embodiments of the present invention;
Fig. 3 is a simplified timing diagram showing events that may occur in the in-vivo sensing system, in accordance with some embodiments of the present invention;
Figs. 4A and 4B are schematic diagrams illustrating a timing diagram of the sensing device, in accordance with some embodiments of the present invention;
Fig. 5A is a schematic flow-chart of a method for concurrent receiving and presenting information gathered by an in vivo sensing device, in accordance with some embodiments of the invention;
Fig. 5B is a schematic flow-chart of a method for real time viewing in-vivo sites, in accordance with some embodiments of the invention; and
Figs. 6-7 are another schematic flow-charts of a method for real time viewing in-vivo sites, in accordance with some embodiments of the invention.

It will be appreciated that for simplicity and clarity of illustration, elements shown in the figures have not necessarily been drawn to scale. For example, the dimensions of some of the elements may be exaggerated relative to other elements for clarity.

### DETAILED DESCRIPTION OF THE INVENTION

In the following detailed description, numerous specific details are set forth in order to provide a thorough understanding of embodiments of the invention. However it will be understood by those of ordinary skill in the art that the embodiments of the invention may be practiced without these specific details. In other instances, well-known methods, procedures, components and circuits have not been described in detail so as not to obscure the embodiments of the invention.

According to some embodiments of the present invention, an in-vivo sensing system may include an in-vivo sensing device, such as, for example, a capsule having image capturing capabilities, and a receiver/recorder, to receive a stream of images from the in-vivo sensing device and to store the stream of images in a memory for a later use. In vivo sensing systems other than capsules may be used.

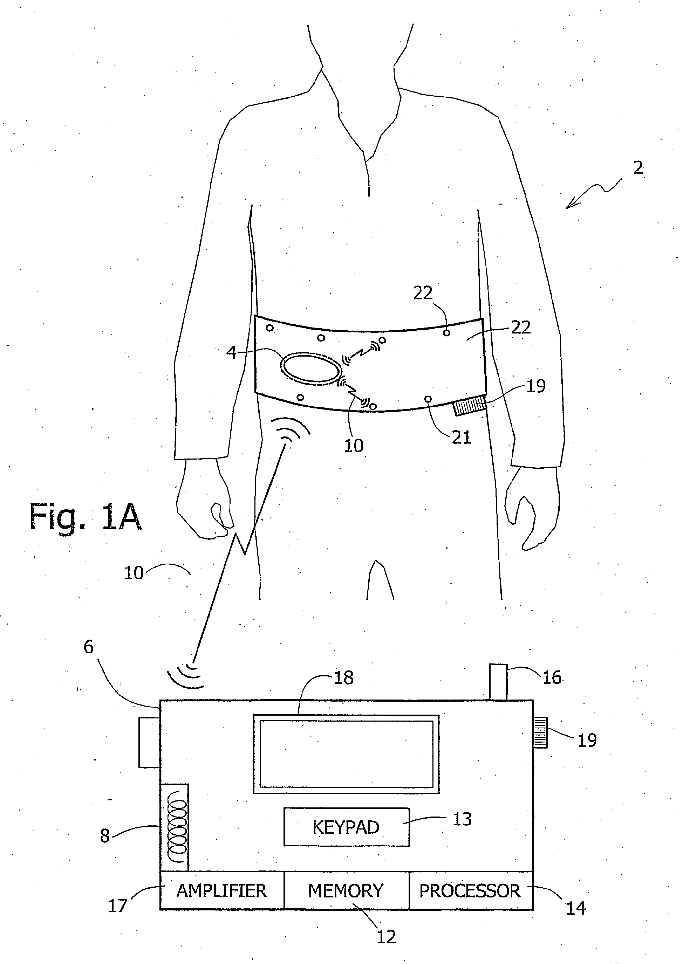

Fig. 1A is a simplified illustration of an exemplary in-vivo sensing system 2, including an in-vivo sensing device 4 and a receiver/recorder 6, in accordance with some embodiments of the invention. According to some embodiments of the invention, sensing device 4 may be a capsule, although other configurations are possible and are under the scope of the invention.

Embodiments of the present invention may be used in conjunction with an in-vivo sensing system or device such as described in US application number 10/046,540. The system according to other embodiments may be used in conjunction with an imaging device and/or receiving system similar to embodiments described in U.S. patent 5,604,531 to Iddan et al. and/or in International Application number WO 01/65995 entitled "A Device And System For In-Vivo Imaging", published on 13 September, 2001.

As illustrated in the following description, sensing device 4 may gather information, such as, for example, images, while inside a patient's body, and may be able to transmit at least that information to receiver/recorder 6 via wireless signals 10 from inside the patient's body. Receiver/recorder 6 may include a memory 12, and/or a buffer and may be able to record information received from sensing device 4 on memory 12. Optionally receiver/recorder 6 may include a display 18 which may include an LCD, TFT, CRT, OLED or other suitable panels. The display may be integrated into receiver/recorder 6 or may be operatively connected to receiver/recorder 6. Receiver/recorder 6 may be able to transfer the received and/or recorded information to display 18 via, for example, a wireless or hard-wired medium.

In some embodiments, receiver/recorder 6 may be or be included in a hand-held or portable device that may include or be connected to an antenna 8. Antenna 8 may be suitable for collecting or transmitting for example wireless signals 10 that may be transmitted from or to device 4 while device 4 is in a patient's body. In some embodiments, receiver/recorder 6 may include one or more processors 14 that may for example encode or de-code wireless signals 10 for display on a display 18, and an amplifier 17 that may be suitable for amplifying a signal 10 received from device 4. In some embodiments, receiver/recorder 6 may be or include for example a personal digital assistant or other hand-held or portable computing device.

In some embodiments, processor 14 may process and/or present information received from receiver/recorder 6 to a human operator while sensing device 4 is still inside the patient's body, and while receiver/recorder 6 is still recording information gathered by sensing device 4. Display 18 unit may include an LCD (Liquid Crystal Display), TFT (Thin Film Transistor), CRT (Cathode Ray Tube) and an OLED (Organic Light Emitting Device) or other suitable panels.

In some embodiments, receiver/recorder 6 may include a control panel such as for example a key pad 13 or keys that may be configured to issue control commands. Such control commands may be encoded or otherwise processed by for example processor 14 to issue commands by wireless signals 10 to device 4.
In some embodiments, such control commands may include for example commands to start or stop imaging, to start or stop collecting samples or other commands to alter a state of one or more functions of device 4.

In operation, receiver/recorder 6 may be held by for example a user or operator on or close to a patient's body and moved around for example an area of the body corresponding to the patient's gastro-intestinal tract. Antenna 8 may be part of or connected to receiver/recorder 6 and may collect an image or other transmitted data when receiver/recorder 6 is passed near or proximate to an area of patient's body where device 4 is located. The area of the patient's body where the image or other data is received may provide a user or operator with an indication of the approximate location of the device 4. The image or other data received by receiver/recorder 6 may provide a further indication of the location of device 4 within an in-vivo area. In some embodiments, based on the location data received by receiver/recorder 6 or the image displayed, a user or other operator may issue a signal 10 to device 4 to activate, deactivate or otherwise change a state of operation of device 4.

In some embodiments, receiver/recorder 6 may be used in addition to an array of antennas 21 that may be worn on a patient's body in for example a belt or garment 22 to record data transmitted from device 4 on a continuous or periodic basis. In some embodiments, receiver/recorder 6 may include a link 19 such as for example a USB, blue-tooth, radio frequency or infra-red link, that may connect to antennas 21 or to a device attached to antennas 21 as may be worn on a patient's body in garment 22. Receiver/recorder 6 may display some or all of the images or other data that may be transmitted by device 4 to antennas 21. In operation, a user may for example wear or carry receiver/recorder 6, such as for example a PDA, cellular phone, having a display or other computing device, and such user may periodically monitor data transmitted or collected by device 4. A user may transmit such data in, for example real time, to a remote operator such as for example a doctor.

In some embodiments, receiver/recorder 6 may include a memory 12 that may be fixed in or removable from receiver/recorder 6. A non-exhaustive list of examples of memory 12 includes any combination of the following: semiconductor devices such as registers, latches, electrically erasable programmable read only memory devices (EEPROM), not AND (NAND) flash memory devices, not OR (NOR) flash memory devices, non-volatile random access memory devices (NVRAM), synchronous dynamic random access memory (SDRAM) devices, RAMBUS dynamic random access memory (RDRAM) devices, double data rate (DDR) memory devices, static random access memory (SRAM), universal serial bus (USB) removable memory, compact flash (CF) memory cards, personal computer memory card international association (PCMCIA) memory cards, security identity module (SIM) cards, MEMORY STICK^{®} cards, and the like; optical devices, such as compact disk read-write memory (CD ROM), and the like; and magnetic devices, such as a hard disk, a floppy disk, a magnetic tape, and the like. In some embodiments memory 12 may hold approximately 10 Gigabytes of memory.

In some embodiments, receiver/recorder 6 may include or be connected to a transmitter such as for example a cellular transmission device 16, that may transmit signals received from device 4 to a remote operator or viewer, or that may receive signals from a remote viewer for further transmission to device 4 by way of for example antenna 8. In some embodiments, the receiver/recorder 6 may download information by way of link 19 and transmit such information to a remote user or store the information in recorder/receiver 6 or in a memory 12 linked to recorder/receiver 6.

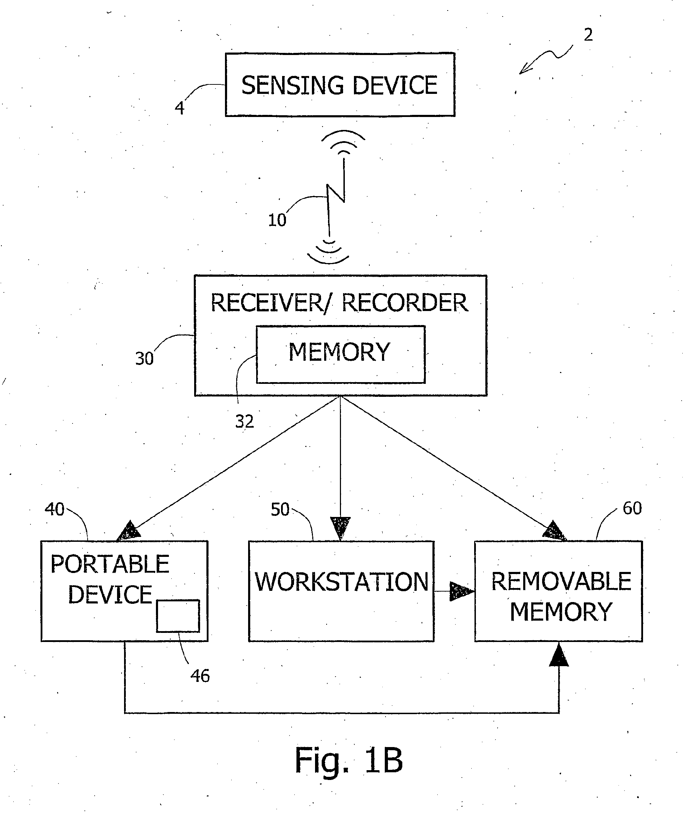

Fig. 1B is a another illustration of an exemplary in-vivo sensing system 2, including for example an in-vivo sensing device 4, a receiver/recorder 30 a portable device 40 such as a notebook or laptop computer, personal digital assistant, and/or a workstation 50 and/or a removable memory 60, in accordance with some embodiments of the present invention.

As illustrated in the following description, sensing device 4 may be able to gather information e.g. raw data, such as a stream of images, while inside a patient's body. According to one embodiment of the present invention, the sensing device 4 may be able to transmit at least that information to a receiver/recorder 30, for example, via a wireless or hard-wired medium 10 while inside the patient's body. According to one embodiment of the present invention, receiver/recorder 30 may include, for example a memory 32, and/or a buffer and may be able to record information received from sensing device 4, for example on memory 32. According to one embodiment of the present invention, the receiver/recorder 6 may be able to transfer the received and/or recorded information to the portable device 40, such as a SONY VAIO™ lightweight belt-portable computer, a personal digital assistant, and/or to the work station 50 via, for example, a wireless or hard-wired medium 44 such as a USB cable, and may be able to do so while receiving/recording information from sensing device 4 and while the sensing device is inside a patient's body.

According to some embodiments of the present invention, the portable device 40 and/or the workstation 50 may be able to process and/or present information e.g. a stream of images, received from receiver/recorder 6, for example, to a human operator while sensing device 4 is still inside the patient's body, and while receiver/recorder 6 is still recording information gathered by sensing device 4. For example, according to one embodiment of the present invention the portable device 40 may include a display unit 46, and may be able to display the stream of images recorded for example in memory 32 on the display unit 46.

Furthermore, according to some embodiments of the present invention, the information may be transmitted from the receiver/recorder 30 and/or may be transferred, for example through the portable device 40 or the workstation 50, to a removable memory 60, such as a DiskonKey or other small and portable memory device. For example the information e.g. stream of images may be recorded by receiver/recorder 30

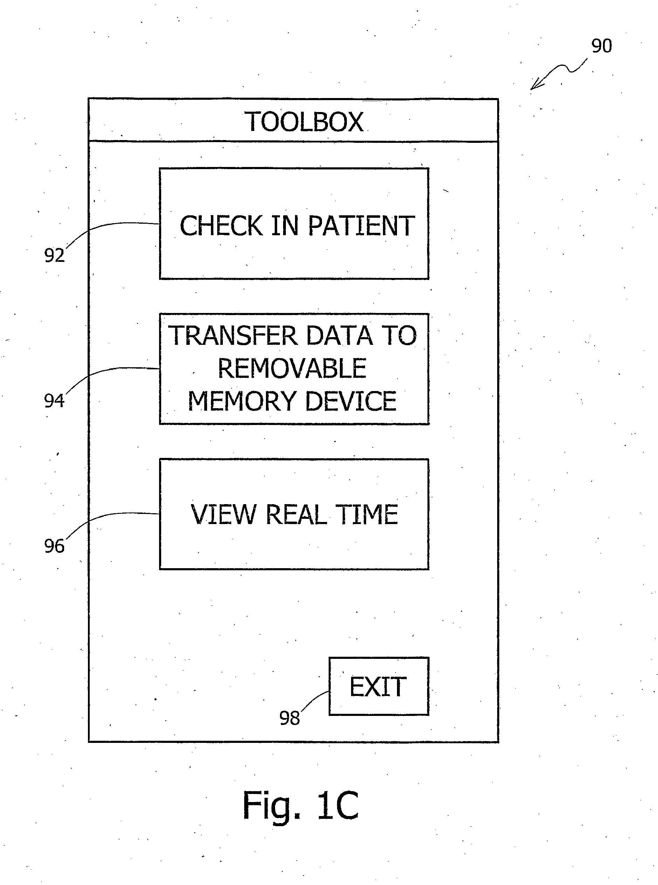

In some embodiments, as shown in Fig. 1C the receiver/recorder 30 and/or the workstation 50 and/or the portable device 40 may include software, operating systems or other instructions that may provide display, analysis and processing capabilities for data or images being transmitted from device 4.

According to some embodiments of the present invention, the operating system may include an information display such as a "Toolbox" screen 90 for performing one or more procedures. For example screen 90 may include a check-in patient box 92, a transfer data to removable memory device box 94 a View Real Time box 96 and an Exit box 98. Other functionality may be included.

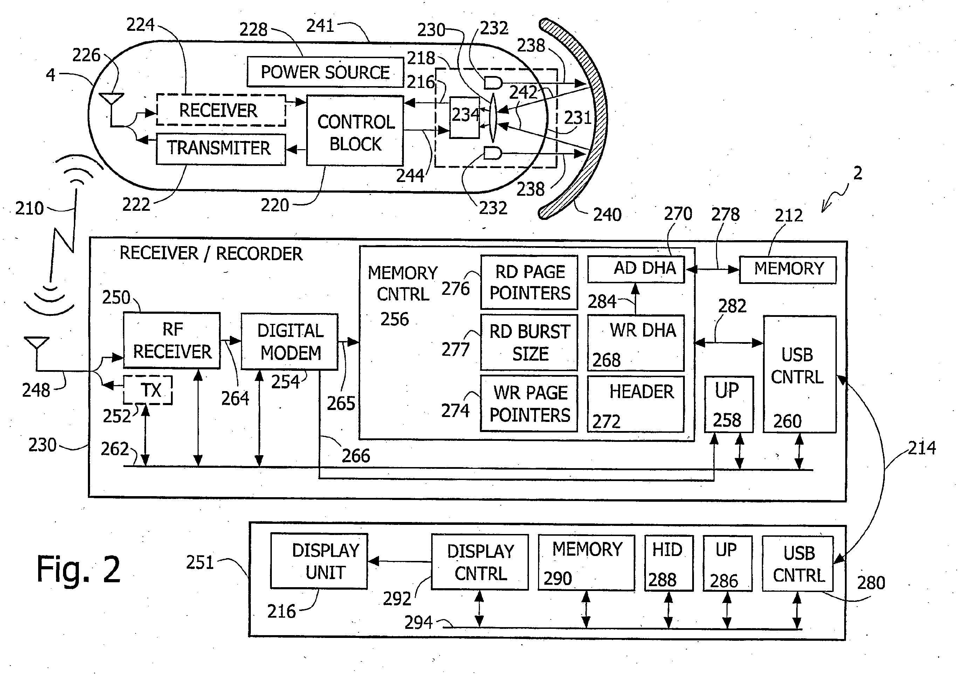

Fig. 2 is an exemplary block-diagram illustration of an in-vivo sensing system 2, in accordance with some embodiments of the present invention. According to some embodiments of the present invention the in-vivo sensing system 2 may include an in-vivo sensing device 4, a receiver/recorder 230 and a portable device 251, which may be or be included in a workstation.

According to some embodiments of the present invention, sensing device 4 may include a container or housing 241. According to one embodiment of the present invention, within the housing 241, may be, for example, an imaging system 218, a control block 220, a transmitter 222, a receiver 224 and an antenna 226. According to one embodiment of the present invention, sensing device 4 may include a power source 228 to provide power to at least imaging system 218, control block 220, transmitter 222, and optional receiver 224.

According to one embodiment of the present invention, all of the components may be sealed within the sensing device 4 body (the body or shell may include more than one piece); for example, an imaging system, power source, and transmitting and control systems, may all be sealed within the sensing device 4 body.

According to some embodiments of the present invention, sensing device 4 typically may be or may include an autonomous swallowable capsule, but device 4 may have other shapes and need not be swallowable or autonomous. Embodiments of device 4 are typically autonomous, and are typically self-contained. For example, device 4 may be a capsule or other unit where all the components are substantially contained within a container or shell, and where device 4 does not require any wires or cables to, for example, receive power or transmit information.

According to some embodiments of the present invention, transmitter 222 may include control capability for, for example controlling the various operations of device 4, although control capability or one or more aspects of control may be included in a separate component.

According to some embodiments of the present invention, power source 228 may include batteries, such as, for example, silver oxide batteries, Lithium batteries, capacitors, or any other suitable power source. In another embodiment of the present invention, power source 228 may not be present and the device may be powered by an external power source, for example, by a magnetic field or electric field that transmits to the device.

Imaging system 218 may include an optical window 230, at least one illumination source 232, such as, for example, a light emitting diode (LED), an OLED (Organic LED) an imaging sensor 234, and an optical system 236.

Imaging sensor 234 may include a solid state imaging sensor, a complementary metal oxide semiconductor (CMOS) imaging sensor, a charge coupled device (CCD) imaging sensor, a linear imaging sensor, a line imaging sensor, a full frame imaging sensor, a "camera on chip" imaging sensor, or any other suitable imaging sensor.

According to some embodiments of the present invention, control block 220 may control, at least in part, the operation of sensing device 4. For example, control block 220 may synchronize time periods, in which illumination source 232 produce light rays, time periods, in which imaging sensor 234 captures images, and time periods, in which transmitter 22 transmits the images. In addition, control block 220 may produce timing signals and other signals necessary for the operation of transmitter 222, optional receiver 224 and imaging sensor 234. Moreover, control block 220 may perform operations that are complimentary to the operations performed by other components of sensing device 4, such as, for example, image data buffering.

According to some embodiments of the present invention, control block 220 may include any combination of logic components, such as, for example, combinatorial logic, state machines, controllers, processors, memory elements, and the like.

Control block 220, transmitter 222, receiver 224 and imaging sensor 234 may be implemented on any combination of semiconductor dies. For example, and although the invention is not limited in this respect, control block 220, transmitter 222 and receiver 224 may be parts of a first semiconductor die, and imaging sensor 234 may be a part of a second semiconductor die. More over, such a semiconductor die may be an application-specific integrated circuit (ASIC) or may be part of an application-specific standard product (ASSP). According to some embodiments dies may be stacked. According to some embodiments some or all of the components may be on the same die.

According to some embodiments of the present invention, illumination source 232 may produce light rays 238 that may penetrate through optical window 231 and may illuminate an inner portion 240 of a body lumen. A non-exhaustive list of examples of a body lumen may include the gastrointestinal (GI) tract, a blood vessel, a reproductive tract, or any other suitable body lumen.

Reflections 242 of light rays 238 from inner portion 240 of a body lumen may penetrate optical window 230 back into sensing device 4 and may be focused by optical system 236 onto imaging sensor 234. According to some embodiments of the present invention, imaging sensor 234 may receive the focused reflections 242, and in response to an image capturing command 244 from control block 220, imaging sensor 234 may capture an image of inner portion 240 of a body lumen. According to some embodiments of the present invention, control block 220 may receive the image of inner portion 240 from imaging sensor 234 over wires 246, and may control transmitter 222 to transmit the image of inner portion 240 through antenna 226 into wireless medium 210.

Sensing device 4 may passively or actively progress along an axis of a body lumen. In time intervals that may or may not be substantially equal and may or may not be related to that progress, control block 220 may initiate capturing of an image by imaging sensor 234, and may control transmitter 222 to transmit the captured image. Consequently, a stream of images of inner portions of the body lumen may be transmitted from sensing device 4 through wireless medium 210.

Device 4 may transmit captured images embedded in "wireless communication frames". A payload portion of a wireless communication frame may include a captured image and may include additional data, such as, for example, telemetry information and/or cyclic redundancy code (CRC) and/or error correction code (ECC). In addition, a wireless communication frame may include an overhead portion that may contain, for example, framing bits, synchronization bits, preamble bits, and the like.

According to some embodiments of the present invention, the receiver/recorder 230 may include an antenna 248, a receiver, such as, for example, RF receiver 250, an optional transmitter (TX) 252, a digital modem 254, a memory controller 256, a processor (uP) 258, and a communication controller, such as, for example, a universal serial bus (USB) controller 260. According to other embodiments of the invention, transmitter 52 may be a unit separate from receiver/recorder 230.

According to some embodiments of the present invention, processor 258 may be able to control the operation of RF receiver 250, optional transmitter 252, digital modem 254, memory controller 256, and USB controller 260 through, for example, a bus 262. In addition, RF receiver 250, optional transmitter 252, digital modem 254, memory controller 256, processor 258 and USB controller 260 may be able to exchange data, such as, for example, images received from sensing device 4, or portions thereof, over bus 262. It may be appreciated, that other methods for control and data exchange are possible, and are under the scope of the invention.

According to some embodiments of the present invention, an antenna 248 may be mounted inside or outside receiver/recorder 230, and both RF receiver 250 and optional transmitter 252 may be coupled to antenna 248. According to some embodiments of the present invention, the transmitter 252 may be able to transmit wireless messages to sensing device 4 through antenna 248. According to some embodiments of the present invention, RF receiver 250 may be able to receive transmissions, such as, for example, a stream of wireless communication frames, from sensing device 4 through antenna 248, and may output signal 264, corresponding to the received wireless communication frames.

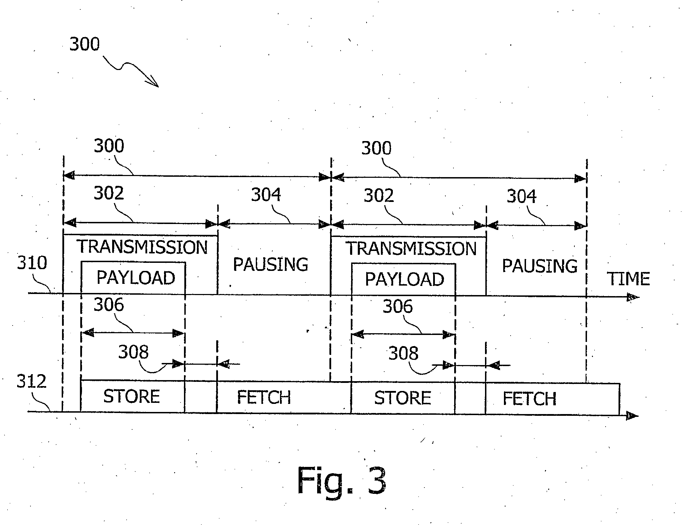

According to some embodiments of the present invention, the digital modem 254 may receive the sampled analog signal bits 264 of RF receiver 250, and may output digital bits 265 that are made from the analog signal 264, and may for example output a payload valid indication 266, that is received by processor 258. According to some embodiments of the present invention, payload valid indication 266 may be asserted by digital modem 254 to, for example, a high logic level, during payload portion (306 of Fig. 3), and may be de-asserted by digital modem 254 to, for example, a low logic level, otherwise. Payload bits 265 may be received by memory controller 256 and payload valid indication 266 may be received by processor 258.

The memory controller 256 may include a write direct memory access (DMA) controller 268, a read DMA controller 270, a header storage 272, a write page pointers storage 274, a read page pointers storage 276 and a read/write burst size storage 277. In response to assertion of payload valid indication 266, processor 258 may store in write page pointers storage 274 pointers to pages in memory 212, and may optionally store a header in header storage 272. In addition, processor 258 may activate write DMA controller 268 to receive payload bits 265 of a wireless communication frame from digital modem 254, and to store the payload bits 265 in memory 212. According to some embodiments of the present invention, the receiver/recorder 230 may communicate with workstation 251 and/or a portable device via medium 214. For example, according to some embodiments of the present invention, receiver/recorder 230 may be able to transfer payloads recorded on memory 212 to workstation 251, and may be able to receive controls from workstation 251. Although the invention is not limited in this respect, medium 214 may be, for example, a USB cable and may be coupled to USB controller 260 of receiver/recorder 230 and to a USB controller 280 of device 251. Alternatively, medium 214 may be wireless, and receiver/recorder 230 and device 251 may communicate wirelessly.

According to some embodiments of the present invention, the receiver/recorder 230 may receive from device 251 via USB controller 260 or another suitable link a control to, for example, start sending a stream of payloads as received from sensing device 4 to device 251, starting at a particular payload of the stream. USB controller 60 may forward the control to processor 58 via bus 62.

According to some embodiments of the present invention, in response to the control received from device 251, processor 258 may program memory controller 256 and USB controller 260 so that read DMA controller 270 fetches payloads from memory 212 in the order requested by device 251, sends the fetched payloads to USB controller 260, and USB controller 260 sends the fetched payloads to device 251. For example, processor 258 may write to read page pointers storage 276 pointers to portions of memory 212 from which read DMA controller 270 may start fetching payloads. In addition, processor 258 may write to read/write burst size storage 277 the number of portions of memory 212 that read DMA controller 270 may fetch in one burst.

The read DMA controller 270 may access memory 212 via memory bus 278 to fetch recorded payloads during times in which write DMA controller 268 does not access memory 212. For at least this purpose, write DMA controller 268 may, for example, output an indication 284 to read DMA controller 270. According to some embodiments of the present invention, the write DMA controller 268 may assert indication 284 to, for example, a high logic level, in response to the assertion of payload valid indication 266, and may de-assert indication 284 to, for example, a low logic level, after completing writing the header to memory 212. According to some embodiments of the present invention, the read DMA controller 270 may start fetching recorded payloads from memory 212 after indication 284 is de-asserted, and may fetch from memory 212 a number of portions equal to the number stored in read/write burst size storage 277.

For example, according to some embodiments of the present invention, the number stored in read/write burst size storage 277 may be related to the number of pointers stored in read page pointers storage 276 and/or to the time available for read DMA controller 270 to fetch recorded payloads from memory 212.

Read DMA controller 270 may send processor 258 an indication over, for example, bus 262, to notify processor 258 of the end of burst.

According to some embodiments moving backwards and forwards in the memory may be enabled. According to other embodiments data may be transmitter (e.g. fetched) directly from the digital modem 254 to the USB controller 260. Thus, writing to read DMA 270 may not be necessary. According to some embodiments read DMA 270 need not be included in the receiver/recorder 230.

Device 251 may include a processor 286, at least one human interface device (HID) 288 such as, for example, a mouse or a keyboard, a memory 290, and a display controller 292 coupled to display unit 216.

According to some embodiments of the present invention, the processor 258 may be able to control the operation of USB controller 280, HID 288, memory 290 and display controller 292 through a bus 294. In addition, USB controller 280, processor 286, HID 288, memory 290 and display controller 292 may be able to exchange data, such as, for example, payloads of wireless communication frames received from receiver/recorder 230, or portions thereof, over bus 294.

According to some embodiments of the present invention, the payloads of wireless communication frames received from receiver/recorder 230 may be transferred from USB controller 280 to memory 290in a DMA process over bus 294, or by way of processor 286.

According to some embodiments of the present invention, the images may be extracted from payloads stored in memory 290 and may be transferred to display unit 216 by way of display controller 292 to be displayed, and/or may be analyzed by processor 286.

A non-exhaustive list of examples of processors 258 and 286 includes a central processing unit (CPU), a digital signal processor (DSP), a reduced instruction set computer (RISC), a complex instruction set computer (CISC) and the like. Moreover, processors 220, 258 and/or 286 may each be part of an application specific integrated circuit (ASIC) or may each be a part of an application specific standard product (ASSP).

A non-exhaustive list of examples of device 251 may include a original equipment manufacturer (OEM) dedicated workstation, a desktop personal computer, a server computer, a laptop computer, a personal digital assistant, a notebook computer, a hand-held computer, and the like.

Reference is made now in addition to FIG. 3, which is a simplified timing diagram showing events that may occur in in-vivo sensing system 2, in accordance with some embodiments of the present invention.

According to some embodiments of the present invention, an exemplary payload of a wireless communication frame transmitted by sensing device 4 may include a captured image of, for example, 256,pixels by 256 pixels and ECC. Sensing device 4 may transmit, for example, two wireless communication frames per second in substantially equal frame time intervals (300) of 500 milliseconds (mS). During a transmission portion (302) of a frame time interval, sensing device 4 may transmit a wireless communication frame, and during a pausing portion (304) of a frame time interval, sensing device 4 may not transmit. In addition, during a payload portion (306) of transmission portion (302), sensing device 4 may transmit the payload of a wireless communication frame, while during the rest of a transmission portion (302), sensing device 4 may transmit the overhead of a wireless communication frame. According to another embodiment a pausing portion (304) may not be needed. Fetching and transmission may be preformed, almost simultaneously during a transmission period (302), as described, for example, below.

According to some embodiments of the present invention, an optional receiver 224 may be able to receive wireless messages via wireless medium 210 through antenna 226, and control block 220 may be able to capture these messages. A non-exhaustive list of examples of such messages includes activating or de-activating image capturing by sensing device 4, controlling the time intervals for capturing images, activating or de-activating transmissions from sensing device 4, or any other suitable messages.

According to some embodiments of the present invention, a write DMA controller 268 may receive payload bits 265, may access memory 212 via a memory bus 278, and may store payload bits 265 in portions of memory 212 pointed to by the pointers stored in write page pointers storage 274. In response to the following de-assertion of payload valid indication 266, processor 258 may send a control to memory controller 256 to indicate the end of the payload bits 265. In response, write DMA controller 268 may append the header stored in header storage 272 to the last byte of the payload bits 265 or before the first byte in memory 212, and may terminate its operation. According to some embodiments of the present invention, as shown in FIG. 3, during a time interval that substantially overlaps payload portion (306), the payload received by RF receiver 250 is stored in memory 212, while during a time interval (308) that is appended to payload portion (306), the header stored in header storage 272 is stored in memory 212.

According to some embodiments of the present invention, the process of processor 258 activating write DMA controller 268 to independently store payload bits 26 in memory 212 may repeat itself for frames of a stream of wireless communication frames. Moreover, the order in which the payloads were received from sensing device 4 may be traceable in memory 212.

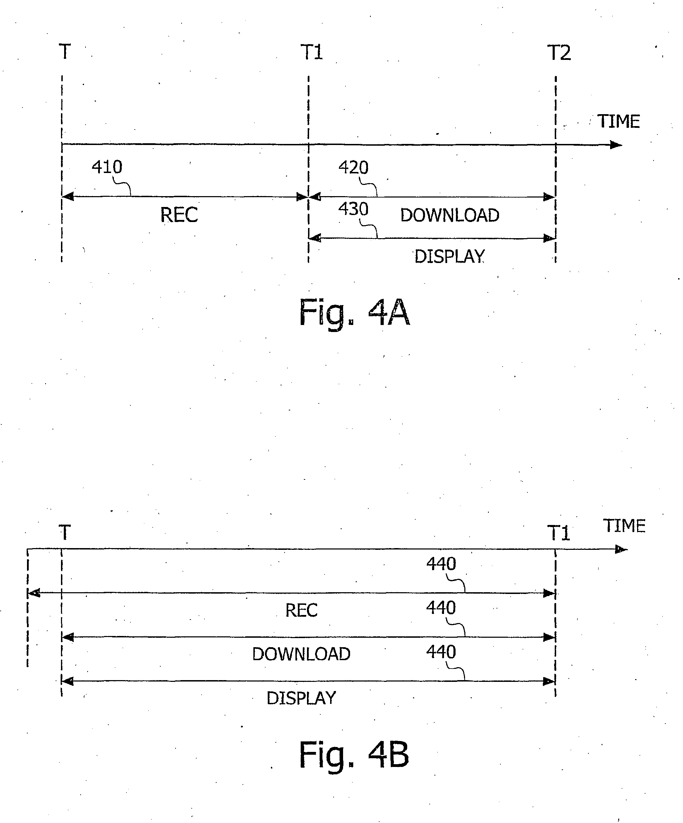

Reference is now made to Figs. 4A and 4B which are schematic diagrams illustrating a timing diagram of the operation of the sensing device, in accordance with some embodiments of the present invention. According to some embodiments of the present invention, as shown in Fig. 4A the total recording period 410, e.g. recording information received from the sensing device 4 by the receiver/recorder 6 into the memory 12, may start at time T and may end at time T₁. A downloading/processing period 420 and a simultaneously displaying period 440, e.g. downloading information from the receiver/recorder 6 to a workstation 50 and displaying the information, may start at time T₁ and may end at time T₂. According to some embodiments, the receiver/recorder 6 may record the information on the memory 12 (recording period 410) and afterwards simultaneously process and/or display the information on a display, such as display 18 (download/process 420 and display 430 periods).

According to some embodiments of the present invention, for example, as shown in Fig. 4B the recording period 440, the download/process period 450 and the display period 460 may all begin at time T and may end at time T₁. For example, the in-vivo sensing system may concurrently record, download and display the information e.g. stream of images, thus enabling a real time viewing of a patient's lumens while the sensing device is inside the patient's body.

According to one embodiment of the present invention, the receiver/recorder 6 may simultaneously record information received from the sensing device 4 on a memory, such as memory 12, process the information and display the information on a display, such as display 18 (as shown in Fig. 1A).

According to another embodiment of the present invention, the receiver/recorder 6 may simultaneously record the information received from the sensing device 4 on a memory, such as memory 32, download the information from the memory to a workstation 50 or to a portable device 40. The information may be simultaneously displayed, during the downloading /processing period 450 and the recording period 440 on a display, such as display 46.

According to some embodiments of the present invention, a control button may be included in the display, such as display 18, that may allow a user to for example, fast-forward, rewind, stop play or reach the beginning or end of , for example, a stream of images, in real time, while the sensing device is still in a patient's body, and during the recording period 440 and downloading/processing period 450.

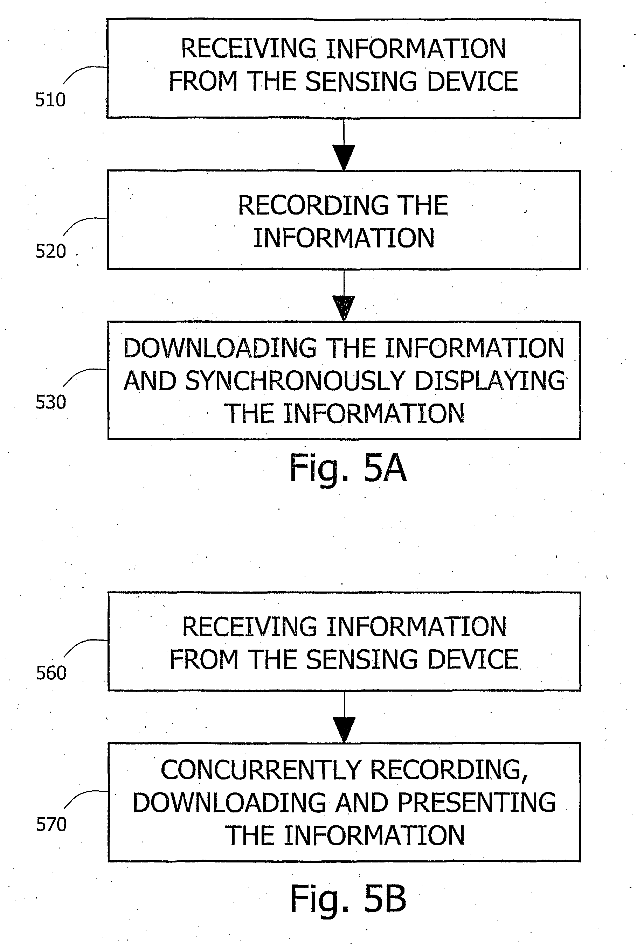

Fig. 5A is a schematic flow-chart of a method for concurrent receiving and presenting information, for example a stream of images, gathered by an in vivo sensing device, in accordance with some embodiments of the invention. In step 510 information, e.g. a stream of images, may be received from the sensing device 4, for example by using the receiver/recorder 6, while the sensing device 4 is inside a patient's body. In step 520 the information may be recorded by the receiver/recorder 6, for example on memory 12.In step 530 the information may be processed by the receiver/recorder and/or may be downloaded, for example from the memory 12 to a workstation and may synchronously be displayed for example on the display 46 (as shown, for example, in Fig. 1B).

Fig. 5B is a schematic flow-chart of a method for real time viewing of in-vivo sites, in accordance with some embodiments of the invention. In step 560 the information may be received from the sensing device, for example by using a receiver/recorder 6. In step 570 the information which was transmitted by the sensing device may be concurrently recorded on a memory, processed by the receiver/recorder and/or downloaded from the memory to, for example a workstation 50. The information may be displayed, for example on display 46, while the receiver/recorder 6 is recording, processing or downloading the information, thus enabling a real time viewing while the sensing device 4 is inside a patient's body.

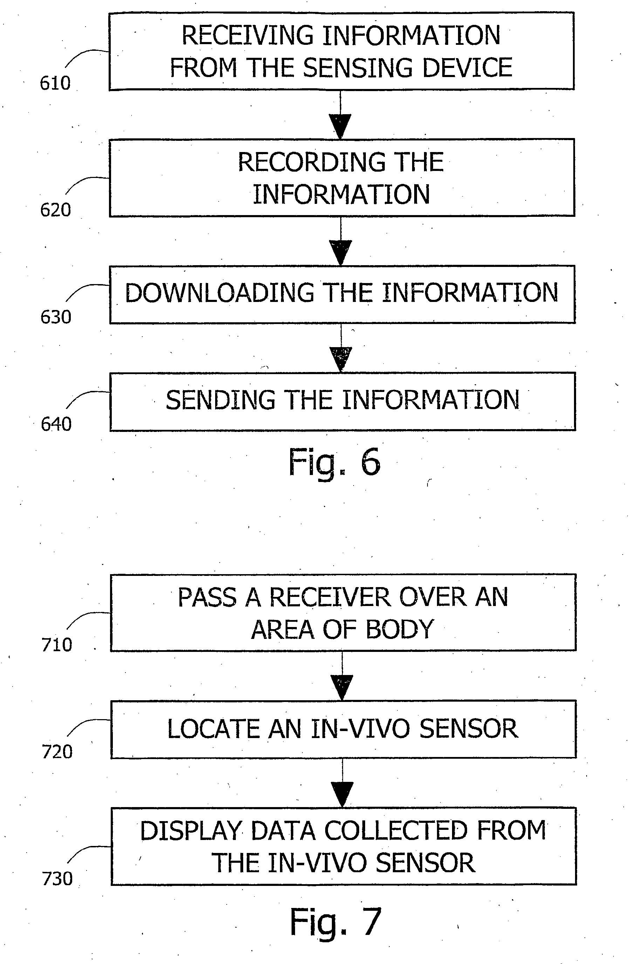

Fig. 6 is an exemplary simplified schematic flow-chart illustration of a method for receiving information in accordance with some embodiments of the invention. In step 610 the information, e.g. a stream of images, may be received from the sensing device 4, for example by using the receiver/recorder 30, while the sensing device 4 is inside a patient's body. In step 620 the information may be recorded, for example using the receiver/recorder 6, on a memory, such as memory 32 (as shown in Fig. 1B). In step 630 the information may be downloaded for example from the memory 32 to a removable memory such as a 5G DiskonKey. According to some embodiments, the information may be concurrently recorded, for example by the receiver/recorder 30 and downloaded for example by the removable memory 60. In step 640 the downloaded information may be sent, for example, with patient check-in information or other information to a central site for processing. The central site may be for example, a hospital or a reading center with health professionals that may be trained to review data acquired from an in-vivo sensing device 4.

Fig. 7 is an exemplary simplified schematic flow-chart illustration of a method for receiving data in accordance with some embodiments of the invention. In step 710, a user or operator may pass a receiver/recorder over an area of a body corresponding to for example a gastro-intestinal tract or other area where an in-vivo sensor may be operating in a patient's body, e.g., in the patient's gastrointestinal tract.

In step 720 the approximate location of an in-vivo sensor may be determined for example based on the location of the receiver/recorder when a signal from the sensor is received by the receiver/recorder. For example, a user may pass the receiver/recorder over the abdomen of the patient, and stop when images appear on a display. Location determination need not be used in some embodiments.

In step 730, a receiver/recorder may display images or other information that was received from the in-vivo sensor.

In some embodiments, images may be recorded or transmitted from the receiver/recorder to a memory or to a remote user by way of, for example, cellular or other magnetic waves.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the scope of the invention.

## Claims

1. An in-vivo sensing system comprising:
an in-vivo sensing device (4) comprising:
an illumination source (232) to illuminate a site in vivo,
an imaging sensor (234) to capture images of the site,
a transmitter (222) to transmit a stream of images,
a control block (220) to synchronize between time periods selected from the group consisting of: time periods in which the illumination source produces light rays, time periods in which imaging sensor captures images, and time periods in which the transmitter transmits images and to perform image data buffering, and
a receiver (224) to wirelessly receive control messages for the control block;
a data recorder (230, Fig. 2; 6, Fig. 1A) external to the in-vivo sensing device (4), wherein the data recorder (230, 6) is configured to receive (210, 10) the stream of images from the sensing device (4); and
a portable device (251, Fig. 2; 6, Fig. 1A) configured to control the data recorder to select images from the received images in a selected order requested by the portable device and to wirelessly download the selected images to said portable device in the selected order while the data recorder (230) is receiving (210) other images of the stream of images, said portable device comprising a display (216, Fig. 2; 18, Fig. 1A) to display the downloaded images while the sensing device (4) is inside the patient's body.

2. The system as in claim 1, wherein the data recorder (230, 6) is configured to simultaneously record images received from the in-vivo sensing device (4) into a memory, process the images, and display the images on the display (216, 18).

3. The system as in claim 1, wherein the data recorder (230, 6) is configured to record images received from the in-vivo sensing device into a memory, and simultaneously process and display the images on the display (216, 18).

4. The system as in claim 1, comprising a workstation and a workstation's display.

5. The system as in claim 4, wherein the data recorder is configured to simultaneously record images of a stream of images received from the in-vivo sensing device into a memory, download the images from the memory to the workstation, and display the images on the workstation's display.

6. The system as in claim 4, wherein the data recorder (230) is configured to record images of a stream of images received from the in-vivo sensing device into a memory, and simultaneously download the images from the memory to the workstation, and display the images on the workstation's display.

7. The system as in claim 4, wherein the data recorder (230) is configured to receive control messages from the workstation.

8. The system as in claim 1, wherein the receiver is configured to wirelessly receive control messages selected from the group consisting of: activating or de-activating image capturing by the sensing device, controlling the time periods for capturing images, and activating or de-activating transmissions from the sensing device.

9. The system as in claim 1, wherein the data recorder (230) is portable.

10. The system as in claim 1, comprising a portable memory, wherein the data recorder (230) is configured to record images received from the in-vivo sensing device into the portable memory.

11. The system as in claim 1, wherein the portable device (251) includes the data recorder (230).

12. A receiver for receiving and recording, in real time, information received from an in-vivo sensing device, comprising:
a memory (12),
an antenna (16, 248) configured to receive a stream of images transmitted from the in-vivo sensing device; and
a processor (14, 256) configured to be controllable by a control received from a portable device (251) to select images and an order in which the selected images are to be downloaded from the receiver to the portable device, and to wirelessly download the selected images to the portable device in the selected order while the receiver is receiving other images of the stream of images, wherein the images are to be displayed on a display (18, 216) of the portable device while the in-vivo sensing device is inside the patient's body.

13. The receiver as in claim 12, comprising a cellular transmission device.

14. A method for real time viewing of an in-vivo site, the method comprising:
- with an in-vivo sensing device (4):
illuminating a site in vivo,
capturing images of the site,
transmitting a stream of images,
synchronizing between time periods selected from the group consisting of: time periods in which light rays are produced for illumination, time periods in which images are captured, and time periods in which images are transmitted, and
performing image data buffering, and
wirelessly receiving control messages for the control block;
- with a data recorder (230, Fig. 2; 6, Fig. 1A) external to the in-vivo sensing device (4):
receiving (210, 10) the stream of images from the sensing device (4); and
- with a portable device (251, Fig. 2; 6, Fig. 1A):
controlling the data recorder to select images from the received images in a selected order requested by the portable device,
wirelessly downloading the selected images to said portable device in the selected order while the data recorder (230) is receiving (210) other images of the stream of images, and
displaying the downloaded images while the sensing device (4) is inside the patient's body.

## Patentansprüche

1. In-Vivo-Sensorsystem, das umfasst:
eine In-Vivo-Erfassungsvorrichtung (4), welche umfasst:
eine Beleuchtungsquelle (232) zum Beleuchten einer Stelle in vivo,
einen Bildgebungssensor (234) zum Aufnehmen von Bildern der Stelle,
einen Sender (222) zum Senden eines Stroms von Bildern,
einen Steuerblock (220) zum Synchronisieren zwischen Zeitperioden, die aus der Gruppe ausgewählt sind, welche besteht aus: Zeitperioden, in welchen die Beleuchtungsquelle Lichtstrahlen erzeugt, Zeitperioden, in welchen der Bildgebungssensor Bilder aufnimmt, und Zeitperioden, in welchen der Sender Bilder sendet, und zum Durchführen von Bilddatenpufferung, und
einen Empfänger (224) zum drahtlosen Empfangen von Steuernachrichten für den Steuerblock;
einen Datenrekorder (230, Fig. 2; 6, Fig. 1A) extern zur In-Vivo-Erfassungsvorrichtung (4), wobei der Datenrekorder (230, 6) konfiguriert ist, den Strom von Bildern aus der Erfassungsvorrichtung (4) zu empfangen (210, 10); und
eine tragbare Vorrichtung (251, Fig. 2; 6, Fig. 1A), welche konfiguriert ist, den Datenrecorder zu steuern, Bilder aus den empfangenen Bildern in einer ausgewählten Reihenfolge auszuwählen, die von der tragbaren Vorrichtung verlangt wird, und die ausgewählten Bilder drahtlos an die tragbare Vorrichtung in der ausgewählten Reihenfolge herunter zu laden, während der Datenrekorder (230) andere Bilder des Stroms von Bildern empfängt (210), wobei die tragbare Vorrichtung eine Anzeige (216, Fig. 2; 18, Fig. 1A) umfasst, um die herunter geladenen Bilder anzuzeigen, während die Erfassungsvorrichtung (4) innerhalb des Patientenkörpers ist.

2. System gemäß Anspruch 1, wobei der Datenrekorder (230, 6) konfiguriert ist, simultan aus der In-Vivo-Erfassungsvorrichtung (4) empfangene Bilder in einem Speicher aufzuzeichnen, die Bilder zu prozessieren und die Bilder auf der Anzeige (216, 18) anzuzeigen.

3. System gemäß Anspruch 1, wobei der Datenrekorder (230, 6) konfiguriert ist, aus der In-Vivo-Erfassungsvorrichtung empfangene Bilder in einem Speicher aufzuzeichnen und simultan die Bilder zu prozessieren und auf der Anzeige (216, 18) anzuzeigen.

4. System gemäß Anspruch 1, umfassend eine Workstation und eine Workstation-Anzeige.

5. System gemäß Anspruch 4, wobei der Datenrekorder konfiguriert ist, simultan Bilder eines Stroms von Bildern, die aus der In-Vivo-Erfassungsvorrichtung empfangen werden, in einem Speicher aufzuzeichnen, die Bilder aus dem Speicher zur Workstation herunter zu laden und die Bilder auf der Workstation-Anzeige anzuzeigen.

6. System gemäß Anspruch 4, wobei der Datenrekorder (230) konfiguriert ist, Bilder eines aus der In-Vivo-Erfassungsvorrichtung empfangenen Stroms von Bildern in einem Speicher aufzuzeichnen und simultan die Bilder aus dem Speicher zu der Workstation herunter zu laden und die Bilder auf der Workstation-Anzeige anzuzeigen.

7. System gemäß Anspruch 4, wobei der Datenrekorder (230) konfiguriert ist, Steuernachrichten aus der Workstation zu empfangen.

8. System gemäß Anspruch 1, wobei der Empfänger konfiguriert ist, Steuernachrichten drahtlos zu empfangen, die ausgewählt werden aus der Gruppe, welche besteht aus: Aktivieren oder Deaktivieren von Bildaufnahme durch die Erfassungsvorrichtung, Steuern der Zeitperioden zum Aufnehmen von Bildern und Aktivieren oder Deaktivieren von Übertragungen aus der Erfassungsvorrichtung.

9. System gemäß Anspruch 1, wobei der Datenrekorder (230) tragbar ist.

10. System gemäß Anspruch 1, umfassend einen tragbaren Speicher, wobei der Datenrekorder (230) konfiguriert ist, aus der In-Vivo-Erfassungsvorrichtung empfangene Bilder im tragbaren Speicher aufzuzeichnen.

11. System gemäß Anspruch 1, wobei die tragbare Vorrichtung (251) den Datenrekorder (230) enthält.

12. Empfänger zum Empfangen und Aufzeichnen, in Echtzeit, von aus einer In-Vivo-Erfassungsvorrichtung empfangener Information, umfassend:
einen Speicher (12),
eine Antenne (16, 248), die konfiguriert ist, einen aus der In-Vivo-Erfassungsvorrichtung gesendeten Strom von Bildern zu empfangen; und
einen Prozessor (14, 256), der konfiguriert ist, durch eine aus einer tragbaren Vorrichtung (251) empfangene Steuerung, Bilder und eine Reihenfolge auszuwählen, in welcher die ausgewählten Bilder aus dem Empfänger an die tragbare Vorrichtung herunter zu laden sind, und die ausgewählten Bilder an die tragbare Vorrichtung in der ausgewählte Reihenfolge herunter zu laden, während der Empfänger andere Bilder des Stroms von Bildern empfängt, wobei die Bilder auf einer Anzeige (18, 216) der tragbaren Vorrichtung anzuzeigen sind, während die In-Vivo-Erfassungsvorrichtung innerhalb des Patientenkörpers ist.

13. Empfänger gemäß Anspruch 12, umfassend eine Zell-Übertragungsvorrichtung.

14. Verfahren zur Echtzeitbetrachtung einer In-Vivo-Stelle, wobei das Verfahren umfasst:
mit einer In-Vivo-Erfassungsvorrichtung (4):
Beleuchten einer Stelle in vivo,
Aufnehmen von Bildern der Stelle,
Senden eines Stroms von Bildern,
Synchronisieren zwischen Zeitperioden, welche ausgewählt sind aus der Gruppe, die besteht aus: Zeitperioden, in denen Lichtstrahlen zur Beleuchtung erzeugt werden, Zeitperioden, in denen Bilder aufgenommen werden, und Zeitperioden, in denen die Bilder gesendet werden, und Durchführen von Bilddatenpufferung, und
drahtloses Empfangen von Steuernachrichten für den Steuerblock;
mit einem Datenrekorder (230, Fig. 2; 6, Fig. 1A) extern zu der In-Vivo-Erfassungsvorrichtung (4):
Empfangen (210, 10) des Stroms von Bildern aus der Erfassungsvorrichtung (4); und
mit einer tragbaren Vorrichtung (251, Fig. 2; 6, Fig. 1A):
Steuern des Datenrekorders zu Auswahlbildern aus den empfangenen Bildern in einer durch die tragbare Vorrichtung verlangten, ausgewählten Reihenfolge,
drahtloses Herunterladen der ausgewählten Bilder auf die tragbare Vorrichtung in der ausgewählten Reihenfolge, während der Datenrekorder (230) andere Bilder des Stroms von Bildern empfängt (210), und
Anzeigen der herunter geladenen Bilder, während die Erfassungsvorrichtung (4) innerhalb des Patientenkörpers ist.

## Revendications

1. Système de détection in vivo comprenant :
un dispositif de détection in vivo (4) comprenant :
une source d'éclairage (232) pour éclairer un site in vivo,
un capteur d'imagerie (234) pour capturer des images du site,
un émetteur (222) pour transmettre un flux d'images,
un bloc de commande (220) pour synchroniser entre des périodes de temps sélectionnées dans le groupe constitué : de périodes de temps dans lesquelles la source d'éclairage produit des rayons lumineux, de périodes de temps dans lesquelles le capteur d'imagerie capture des images et de périodes de temps dans lesquelles l'émetteur transmet des images et pour effectuer une mise en mémoire tampon de données d'image, et
un récepteur (224) pour recevoir sans fil des messages de commande pour le bloc de commande ;
un enregistreur de données (230, Fig. 2 ; 6, Fig. 1A) externe au dispositif de détection in vivo (4), dans lequel l'enregistreur de données (230, 6) est configuré pour recevoir (210, 10) le flux d'images à partir du dispositif de détection (4) ; et
un dispositif portatif (251, Fig. 2 ; 6, Fig. 1A) configuré pour commander l'enregistreur de données afin de sélectionner des images parmi les images reçues dans un ordre sélectionné demandé par le dispositif portatif et pour télécharger sans fil les images sélectionnées sur ledit dispositif portatif dans l'ordre sélectionné pendant que l'enregistreur de données (230) reçoit (210) d'autres images du flux d'images, ledit dispositif portatif comprenant un dispositif d'affichage (216, Fig. 2 ; 18, Fig. 1A) pour afficher les images téléchargées pendant que le dispositif de détection (4) est à l'intérieur du corps du patient.

2. Système selon la revendication 1, dans lequel l'enregistreur de données (230, 6) est configuré pour enregistrer simultanément des images reçues à partir du dispositif de détection in vivo (4) dans une mémoire, pour traiter les images, et pour afficher les images simultanément sur le dispositif d'affichage (216, 18).

3. Système selon la revendication 1, dans lequel l'enregistreur de données (230, 6) est configuré pour enregistrer des images reçues à partir du dispositif de détection in vivo dans une mémoire, et pour traiter et afficher simultanément les images sur le dispositif d'affichage (216, 18).

4. Système selon la revendication 1, comprenant un poste de travail et un dispositif d'affichage du poste de travail.

5. Système selon la revendication 4, dans lequel l'enregistreur de données est configuré pour enregistrer simultanément des images d'un flux d'images reçues à partir du dispositif de détection in vivo dans une mémoire, pour télécharger les images à partir de la mémoire sur le poste de travail, et pour afficher les images sur le poste de travail du dispositif d'affichage.

6. Système selon la revendication 4, dans lequel l'enregistreur de données (230) est configuré pour enregistrer des images d'un flux d'images reçues à partir du dispositif de détection in vivo dans une mémoire, et pour télécharger simultanément les images à partir de la mémoire sur le poste de travail, et pour afficher les images sur le dispositif d'affichage du poste de travail.

7. Système selon la revendication 4, dans lequel l'enregistreur de données (230) est configuré pour recevoir des messages de commande à partir du poste de travail.

8. Système selon la revendication 1, dans lequel le récepteur est configuré pour recevoir sans fil des messages de commande sélectionnés dans le groupe constitué : de l'activation ou la désactivation de la capture d'image par le dispositif de détection, de la commande des périodes de temps pour capturer des images, et de l'activation ou la désactivation des transmissions à partir du dispositif de détection.

9. Système selon la revendication 1, dans lequel l'enregistreur de données (230) est portatif.

10. Système selon la revendication 1, comprenant une mémoire portative, dans lequel l'enregistreur de données (230) est configuré pour enregistrer des images reçues à partir du dispositif de détection in vivo dans la mémoire portative.

11. Système selon la revendication 1, dans lequel le dispositif portatif (251) comporte l'enregistreur de données (230).

12. Récepteur pour recevoir et enregistrer, en temps réel, des informations reçues à partir d'un dispositif de détection in vivo, comprenant :
une mémoire (12),
une antenne (16, 248) configurée pour recevoir un flux d'images transmis par le dispositif de détection in vivo ; et
un processeur (14, 256) configuré pour pouvoir être commandé par une commande reçue à partir d'un dispositif portatif (251) pour sélectionner des images et un ordre dans lequel les images sélectionnées doivent être téléchargées à partir du récepteur sur le dispositif portatif, et pour télécharger sans fil les images sélectionnées sur le dispositif portatif dans l'ordre sélectionné pendant que le récepteur reçoit d'autres images du flux d'images, où les images doivent être affichées sur un dispositif d'affichage (18, 216) du dispositif portatif pendant que le dispositif de détection in vivo est à l'intérieur du corps du patient.

13. Récepteur selon la revendication 12, comprenant un dispositif de transmission cellulaire.

14. Procédé pour la visualisation en temps réel d'un site in vivo, le procédé comprenant :
- avec un dispositif de détection in vivo (4) :
l'éclairage d'un site in vivo,
la capture d'images du site,
la transmission d'un flux d'images,
la synchronisation entre des périodes de temps sélectionnées dans le groupe constitué : de périodes de temps dans lesquelles des rayons lumineux sont produits pour l'éclairage, de périodes de temps dans lesquelles des images sont capturées, et de périodes de temps dans lesquelles des images sont transmises, et l'exécution d'une mise en mémoire tampon de données d'image, et
la réception sans fil de messages de commande pour le bloc de commande ;
- avec un enregistreur de données (230, Fig. 2 ; 6, Fig. 1A) externe à l'appareil de détection in vivo (4) :
la réception (210, 10) du flux d'images à partir du dispositif de détection (4) ; et
- avec un dispositif portatif (251, Fig. 2 ; 6, Fig. 1A) :
la commande de l'enregistreur de données pour sélectionner des images parmi les images reçues dans un ordre sélectionné demandé par le dispositif portatif,
le téléchargement sans fil des images sélectionnées sur ledit dispositif portatif dans l'ordre sélectionné pendant que l'enregistreur de données (230) reçoit (210) d'autres images du flux d'images, et
l'affichage des images téléchargées pendant que le dispositif de détection (4) est à l'intérieur du corps du patient.
